# EUROPEAN PATENT SPECIFICATION

(11) **EP 2 903 532 B1**
(45) Date of publication and mention of the grant of the patent: **17.08.2022**
(21) Application number: 13843189.5
(22) Date of filing: 04.10.2013
(51) Int. Cl.: A61B 8/12, A61B 8/00, A61B 8/08, A61B 8/14, A61B 5/00

(54) **SYSTEM FOR OPERATION OF THREE-DIMESIONAL IMAGING SYSTEMS**
SYSTEME ZUM BETRIEBEN VON DREIDIMENSIONALEN BILDGEBUNGSSYSTEMEN
SYSTÈMES POUR LE FONCTIONNEMENT DE SYSTÈMES D'IMAGERIE TRIDIMENSIONNELLE

(30) Priority: 05.10.2012 US 201261710408 P; 05.10.2012 US 201261710437 P; 05.10.2012 US 201261710432 P
(43) Date of publication of application: 12.08.2015
(73) Proprietor: Kemp, Nathaniel J., Concord, MA 01742 (US); Jones, Jesse, San Diego, CA 92130 (US); Begin, Elizabeth, Billerica, MA 01862 (US); Nair, Anuja, Bedford, MA 01730 (US); Sproul, Jason, Watertown, MA 02472 (US)
(72) Inventor: Kemp, Nathaniel J., Concord, MA 01742 (US); Jones, Jesse, San Diego, CA 92130 (US); Begin, Elizabeth, Billerica, MA 01862 (US); Nair, Anuja, Bedford, MA 01730 (US); Sproul, Jason, Watertown, MA 02472 (US)
(74) Representative: Philips Intellectual Property & Standards
(86) International application number: PCT/US2013/063522
(87) International publication number: WO 2014/055908

(56) References cited:
- WO-A1-2007/107918
- WO-A1-2011/117788
- WO-A1-2012/061940
- WO-A2-2008/038215
- JP-A- 2002 336 253
- US-A1- 2001 031 920
- US-A1- 2006 253 024
- US-A1- 2012 083 696
- Jiechen Yin ET AL: "Novel combined miniature optical coherence tomography ultrasound probe for in vivo intravascular imaging", Journal of biomedical optics, 060505, 1 June 2011 (2011-06-01), XP055280062, United States DOI: 10.1117/1.3589097 Retrieved from the Internet: URL:file:///C:/Users/KL50547/AppData/Local /Microsoft/Windows/Temporary Internet Files/Content.IE5/GYTJAKJ8/060505_1.pdf
- YIN JIECHEN ET AL: "Integrated optical coherence tomography - ultrasound system and miniaturized probes for intravascular imaging", OPTICAL COHERENCE TOMOGRAPHY AND COHERENCE DOMAIN OPTICAL METHODS IN BIOMEDICINE XV, SPIE, 1000 20TH ST. BELLINGHAM WA 98225-6705 USA, vol. 7889, no. 1, 788918, 10 February 2011 (2011-02-10), XP060006378, DOI: 10.1117/12.875128
- LAURA J BRATTAIN ET AL: "Enabling 3D Ultrasound Procedure Guidance through Enhanced Visualization", 27 June 2012 (2012-06-27), INFORMATION PROCESSING IN COMPUTER-ASSISTED INTERVENTIONS, SPRINGER BERLIN HEIDELBERG, BERLIN, HEIDELBERG, PAGE(S) 115 - 124, XP047006735, ISBN: 978-3-642-30617-4

## Description

### Field of the Invention

The invention relates to systems for the operation of three-dimensional imaging systems.

### Background

A number of technologies are available for three-dimensional imaging such as ultrasound and tomography. In medical imaging, for example, an imaging system captures an image from a patient for the analysis of bodily tissue. A physician examining such an image will sometimes recognize irregularities that indicate the presence in the tissue of a medically significant feature such as a plaque on an artery wall. To study this feature in greater detail, the physician uses the system to capture another image that focuses on the feature. To do so, the physician must input parameters to control the image capture operation, such as an indication of what region of the tissue to scan.

Programming scan parameters involves interpreting an image and either inputting numbers or mechanically driving an imaging catheter to an unseen position inside of the tissue.

For example, if a physician scans a vessel with a device that takes an image of a 10 cm range of the vessel and the resulting display reveals a region of interest near an end of the 10 cm range, the physician will study the display and determine by visual inspection approximately where within the tissue the feature is positioned. The physician then inputs numbers (e.g., 7 cm-9 cm) and triggers operation of the system to capture an image of the referenced portion of the vessel. The physician repeats these steps until the desired image is captured.

Some systems require the physician to set up a subsequent image capture operation by mechanically positioning the catheter within the tissue, for example, by repeatedly pressing a button on a handheld control module. These steps take a significant amount of time during which the catheter is resting inserted into a patient' s body.

WO 2012/061940 A1 discloses systems and methods for performing a minimally invasive procedure in an automated or semi-automated fashion, where an imaging probe having an imaging modality compatible with the presence of an intraluminal medium is employed to record images that are processed to identify regions of interest and direct a medium displacement operation during a subsequent minimally invasive operation that benefits from the displacement of the intraluminal medium. The minimally invasive operation includes recording images with a second imaging modality, or may be a therapeutic treatment.

Under either approach, the physician must position the apparatus to a "best guess" position, intended to correspond to the region of interest. These methods of controlling imaging operation require the image capture parameters to be established through operations that have no inherent relationship to the tissue (mechanically driving the catheter to an unseen position or inputting numbers that represent an interpretation of the tissue). Thus, the parameter input steps are a source of inaccuracy and imprecision in the results and capturing a desired image can require expensive and time-consuming iterations and repetitions of the process.

Due to the fact that these procedures are time consuming, particularly where repeated iterations of inspection and parameter input are required, a costly amount of time is required from physicians and attendant staff. Since the parameter setting methods are imprecise, the resulting images are not optimal in that they do not always include the entire region of interest and only that region. Images can also contain artifacts that interfere with the usefulness of the image.

For example, some imaging technologies use a thin, flexible guidewire that is inserted into a lumen to act as a guide for subsequent insertion of the imaging catheter to the target imaging area. Once at the target imaging area, the imaging catheter rotates while moving longitudinally alongside the guidewire to acquire imaging data.

Because the imaging core rotationally sweeps past the guidewire during each rotation, the resulting images typically include a guidewire shadow or artifact that obscures imaging of tissue located behind the guidewire. The guidewire artifact is caused by the inability of the sensing energy, such as light or sound, to adequately penetrate the guidewire. The guidewire artifact is considered to be an unavoidable discontinuity in the imaging data that reduces image quality and can lead to misinterpretation of the resulting medical images.

### Summary

The invention is defined by the claims. It provides systems for establishing control parameters for capturing a three-dimensional image of tissue. Tools of the invention allow an operator of an imaging system to select image capture parameters by interacting with a display of an image that includes a target region to be captured. By selecting image capture parameters from the displayed image, an operator generates parameters that have an inherent relationship to the tissue to be imaged. Because the image capture parameters (such as target image boundary, start and stop positions, contrast, etc.) are inherently related to the target tissue and any region of interest therein, the capture parameters are precise and accurate. Thus, systems of the invention avoid expensive and time-consuming "best guess" approaches to imaging and provide a detailed and accurate image of a region of interest in tissue.

A method of imaging tissue is provided as example, which includes displaying a first image of the tissue and receiving a selection from within that image. The selected points are used to establish a boundary and an imaging system is operated to capture a three-dimensional image of tissue within that boundary. An imaging system can capture the image by translating an imaging device along a line, for example, by pulling or pushing a fiber optic or sonic catheter through a vessel (i.e., inside of the target tissue). An image can be captured by any means known in the art such as, for example, using sound waves or light waves. The image capture system can first capture a first image and display that to an operator, allowing the operator to make a selection. For example, where the image is displayed on a computer monitor, an operator can select pixels within the display using a computer pointing device or an element of a graphical user interface (GUI). By selecting parameters for image capture by interacting with a display that includes an image of the target tissue, parameters are established that inherently relate to the tissue being studied. For example, a region of the tissue can be chosen by mouse clicks within the display or by sliders rendered within a GUI, and that region can be used to establish a start position or stop position for a subsequent image capture operation (e.g., start and stop points for translation of an intralumen catheter). A second three-dimensional image is captured, representing the portion of the tissue indicated by the selection. This second image can then be provided to a user, for example, by displaying it using a display device or writing a file including the image data.

An electronic device for imaging tissue is provided that includes a memory coupled to one or more processors and configured to display a first image of tissue. The electronic device is operable to receive a selection of points from within the first image, establish a boundary corresponding to the selected points, and capture a three-dimensional image of the tissue within the designated boundary. The electronic device captures the three dimensional image through the operation of an image capture device such as an intravascular IVUS or OCT device that operates by translating an image capture device along a line. The electronic device can receive a selection in the form of input generated via use of computer devices, such as peripheral hardware, and in which the input designates a set of pixels within the first image. The input can be generated by a user interacting with the first image, for example, in the context of a graphical user interface rendered by the electronic device. The user makes a selection of a portion of the first image and the electronic device captures a three dimensional image of the tissue for example, by starting or stopping the translation of an intravascular catheter at start point or stop point in a boundary that corresponds to part of the selection.

As example an automatic longitudinal image playback system and method are provided for three dimensional medical imaging. Systems and methods of the example receive a three dimensional data set and display a series of coaxial longitudinal images (i.e., each rotationally offset from another around an axis) in sequence, creating a video effect as if the view were rotating through the tissue. Since the video view plays without simultaneous hands-on operation by a user, a user is free to operate the image capture controls of the system while visually inspecting the subject tissue in three dimensions through the display. Where the tissue includes a feature of interest, the user may establish scan parameters such as a start or stop point while seeing the three dimensional shape, orientation, and extent of the feature in the display. This allows the user to make a high resolution close-up scan that is directed at the feature accurately and with precision.

A method for generating an image of tissue is provided as example, which includes obtaining and storing in a tangible memory coupled to a processor within a computer device comprising a display device a three-dimensional data set representing tissue, composing a plurality of two-dimensional coaxial images from the three dimensional image file that each represent a planar region of the tissue, and displaying via the display device the two-dimensional images sequentially. By presenting the two-dimensional images sequentially, in an order such that each is progressively more rotationally offset around the axis, a video is created in which a cross-sectional view rotates through the imaged tissue. This way, a video can be presented of any three-dimensional image of tissue, such as, for example, IVUS or OCT images, which may include data in the format of a helical array of scan lines.

A video can be provided based on a user' s input into a system as obtained, for example, through their interaction with a graphical user interface (GUI). A user can choose points within the three-dimensional image set at which the video should begin and end, for example, by positioning indicator bars within a view that is substantially orthogonal to the video image view. A GUI can further provide tools with which a user can control a video view such as by starting, pausing, stopping, fast forwarding, reversing, or toggling a video to full screen. In certain examples, methods and devices are provided for automatically playing a video including a rotation through a cross section of a tissue image, for example, responsive to capturing a data set with a medical imaging system.

A device for generating an image of tissue is provided as example, that includes a memory coupled to a processor and a display device, in which the processor is configured to obtain a three-dimensional data set representing tissue, compose a plurality of two-dimensional coaxial images from the three dimensional image file and representing a planar region of the tissue, and display the two-dimensional images sequentially on the display device.

The processor in the device preferably presents a GUI allowing a user to interact with images and establish parameters for video playback. Based on user input, the processor controls video playback. In certain embodiments, a device of the invention is operably coupled to a three dimensional imaging system such as an IVUS or OCT medical imaging system. A device is provided to create a video showing a slice of tissue in which the slice rotates around an axis, thereby providing three dimensional information about the tissue. In some embodiments, a video plays automatically, responsive to image capture by a medical imaging system or user input.

Described examples generally improve image quality of tomographic imaging systems that use a rotating imaging probe in parallel with an object, such as a guidewire, that creates a discontinuity in an imaging data set. Through use of the image processing techniques, discontinuities, such as guidewire artifacts, are removed from an imaging data set, and thus removed from any resulting images. The resulting images with the artifacts removed include B-scans or scan-converted images. This advantageously reduces any error associated with misinterpretation of the guidewire artifact in the resulting images and overall improves image quality.

Guidewire artifacts and other artifacts can be removed from images acquired from any tomographic imaging system that utilizes a rotating probe, including, for example, optical coherence tomography, ultrasound technology, intravascular spectroscopy, or photo-acoustic tomography. The medical images acquired with rotating probes are typically intraluminal images taken within a biological lumen, such as a blood vessel and an intestine.

A guidewire artifact is removed by acquiring at least two images an imaging surface in which image is formed from a set of imaging data. The imaging surface can include tissue, stents, plaque, etc. The guidewire artifact present in one of the at least two images is detected, and then replaced with data representing the imaging surface obtained from at least one other image.

The at least two images of the imaging surface are acquired by repeated imaging of the same imaging surface. For catheter imaging, the images are acquired by pulling back or pushing forward the imaging catheter across the same region of the imaging surface within a lumen. During image acquisition, the guidewire is rotated or moved to a different position within the lumen so that the at least two images are of the same imaging surface with the guidewire in a different location.

The guidewire artifact is then detected in at least one of the images. The guidewire artifact can be automatically or manually detected in an image. After the guidewire artifact is detected within one of the at least two images, the guidewire artifact is replaced with data representing the imaging surface at the same location obtained from another one of the at least two images.

Prior to detection of the guidewire artifact, the acquired images are registered to obtain the Cartesian coordinates for the imaging data set of each image.
Registration allows for data from each image to be compared and integrated. The images can be manually or automatically registered using, for example, a phase correlation technique. By detecting the guidewire artifact, the position and the size of the guidewire within the Cartesian coordinate system is determined. Because the guidewire is moved during imaging, the guidewire artifact present in one of the images shares the same Cartesian coordinates as the imaging surface obtained from another image of the at least two images. Data representing the detected guidewire artifact is then replaced with data representing the imaging surface sharing the same Cartesian coordinates with the guidewire from another one of the at least two images. In one aspect, the resulting image with the guidewire artifact removed is interpolated to improve image quality.

Other and further aspects and features of the invention will be evident from the following detailed description and accompanying drawings, which are intended to illustrate, not limit, the invention.

### Brief Description of the Drawings

FIG. 1 is a perspective view of a vessel.
FIG. 2 is a cross-sectional view of the vessel shown in FIG. 1.
FIG. 3 is a diagram of components of an optical coherence tomography (OCT) system.
FIG. 4 is a diagram of the imaging engine shown in FIG. 3.
FIG_{.} 5 is a diagram of a light oath in an OCT system.
FIG. 6 is a patient interface module of an OCT system.
FIG. 7 is an illustration of the motion of parts of an imaging catheter.
FIG. 8 shows an array of A scan lines of a three-dimensional imaging system .
FIG. 9 shows the positioning of A scans within a vessel.
FIG. 10 illustrates a set of A scans used to compose a B scan.
FIG. 11 shows the set of A scans shown in FIG. 10 within a cross section of a vessel.
FIG. 12A shows a longitudinal plane through a vessel including several A scans.
FIG. 12B shows the longitudinal plane of FIG. 12A without the A scans.
FIG. 13 is a perspective view of an image longitudinal display (ILD) in the same perspective as the longitudinal plane shown in FIGS. 12 and 12A.
FIG. 14 illustrates a portion of a vessel.
FIG. 15 is a cross-sectional view of the vessel shown in FIG. 14.
FIG. 16A is an illustration of a display including an image of the vessel shown in FIGS. 14-15.
FIG. 16B shows an ultrasound display including an image of the vessel shown in FIGS. 14-15.
FIG. 17 is an illustration of a display including parameter-setting markers shown on the ILD.
FIG. 18 is an illustration of the display shown in FIG. 17 in which the markers have been positioned to represent a selection of points within the ILD in the display.
FIG. 19 is an illustration of a display including an image of the portion of the vessel corresponding to the selection represented by the position of the markers shown in FIG. 18.
FIG. 20 is a system diagram according to certain embodiments.
FIG. 21 is an illustration of a display including selection tools.
FIGS. 22A-22C show a series of displays that present a video.
FIG. 23 is a two-window display.
FIG. 24 is a video player display
FIG. 25 shows a video interface in a web browser.
FIG. 26 shows a sample OCT B-Scan image calculated from 660 A-scans.
FIG. 27 shows a scan-converted OCT image from the B-scan of Figure 14.
FIG. 28 shows an OCT B-scan image with a guidewire artifact and shadow highlighted.
FIG. 29 shows a scan-converted OCT image with a guidewire artifact and shadow highlighted.
FIG. 30 depicts a block diagram for guidewire artifact removal according to an example.

### Detailed Description

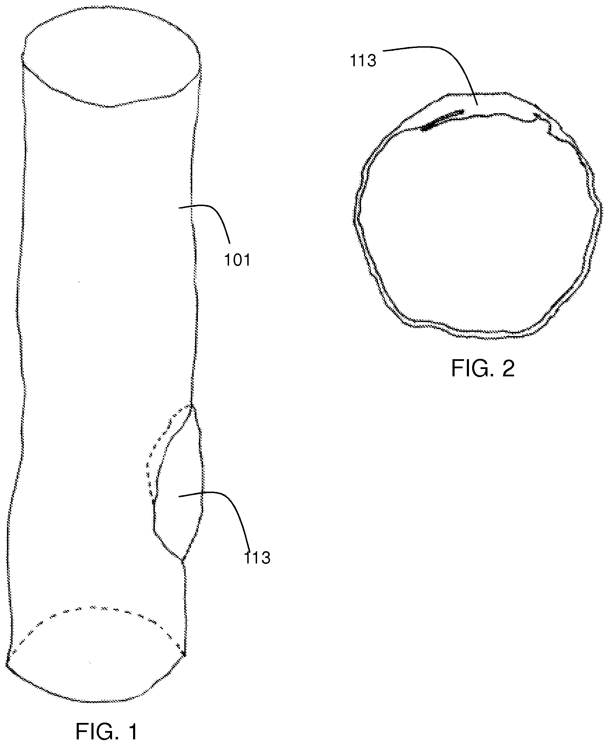

The invention provides systems for establishing control parameters for capturing a three-dimensional image of tissue. The invention provides systems for setting a parameter for capturing a three-dimensional image of tissue based on an existing image. Systems of the invention have application in intravascular imaging methodologies such as intravascular ultrasound (IVUS) and optical coherence tomography (OCT) among others that produce a three-dimensional image of a vessel. A segment of a vessel 101 is shown in FIG. 1 having a feature 113 of interest. FIG. 2 shows a cross-section of vessel 101 through feature 113. Intravascular imaging involves positioning an imaging device near feature 113 and collecting data representing a three-dimensional image.

Any three-dimensional imaging system may be used in systems and methods for example, IVUS; magnetic resonance imaging; elastographic techniques such as magnetic resonance elastography or transient elastography systems such as FibroScan by Echosens (Paris, France); electrical impedance tomography; and OCT. Systems and method include processing hardware configured to interact with more than one different three dimensional imaging system so that the tissue imaging devices and methods described herein can be alternatively used with OCT, IVUS, or other hardware.

Any target can be imaged by methods of the examples and by systems of the invention including, for example, bodily tissue. In certain embodiments, systems of the invention image within a lumen of tissue. Various lumen of biological structures may be imaged including, but not limited to, blood vessels, vasculature of the lymphatic and nervous systems, various structures of the gastrointestinal tract including lumen of the small intestine, large intestine, stomach, esophagus, colon, pancreatic duct, bile duct, hepatic duct, lumen of the reproductive tract including the vas deferens, vagina, uterus and fallopian tubes, structures of the urinary tract including urinary collecting ducts, renal tubules, ureter, and bladder, and structures of the head and neck and pulmonary system including sinuses, parotid, trachea, bronchi, and lungs.

In an exemplary embodiment, the invention provides a system for capturing a three dimensional image by OCT. Commercially available OCT systems are employed in diverse applications such as art conservation and diagnostic medicine, e.g., ophthalmology. OCT is also used in interventional cardiology, for example, to help diagnose coronary artery disease. OCT systems and methods are described in U.S. Pub. 2011/0152771; U.S. Pub. 2010/0220334; U.S. Pub. 2009/0043191; U.S. Pub. 2008/0291463; and U.S. Pub. 2008/0180683.

In OCT, a light source delivers a beam of light to an imaging device to image target tissue. Within the light source is an optical amplifier and a tunable filter that allows a user to select a wavelength of light to be amplified. Wavelengths commonly used in medical applications include near-infrared light, for example between about 800 nm and about 1700 nm.

Generally, there are two types of OCT systems, common beam path systems and differential beam path systems, that differ from each other based upon the optical layout of the systems. A common beam path system sends all produced light through a single optical fiber to generate a reference signal and a sample signal whereas a differential beam path system splits the produced light such that a portion of the light is directed to the sample and the other portion is directed to a reference surface. Common beam path interferometers are further described for example in U.S. Pat. 7,999,938; U.S. Pat. 7,995,210; and U.S. Pat. 7,787,127.

In a differential beam path system, amplified light from a light source is input into an interferometer with a portion of light directed to a sample and the other portion directed to a reference surface. A distal end of an optical fiber is interfaced with a catheter for interrogation of the target tissue during a catheterization procedure. The reflected light from the tissue is recombined with the signal from the reference surface forming interference fringes (measured by a photovoltaic detector) allowing precise depth-resolved imaging of the target tissue on a micron scale. Exemplary differential beam path interferometers are Mach-Zehnder interferometers and Michelson interferometers. Differential beam path interferometers are further described for example in U.S. Pat. 7,783,337; U.S. Pat. 6,134,003; and U.S. Pat. 6,421,164.

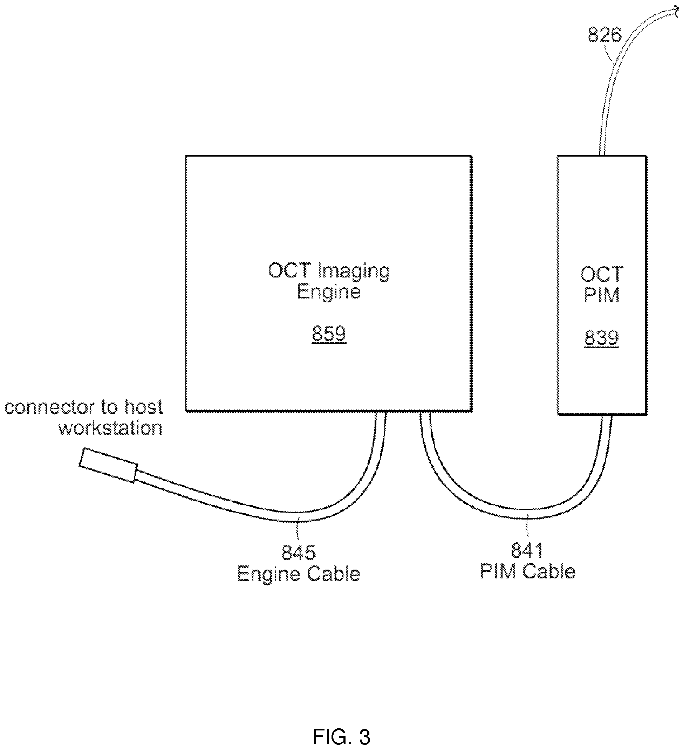

In certain embodiments, the invention provides a differential beam path OCT system with intravascular imaging capability as illustrated in FIG. 3. For intravascular imaging, a light beam is delivered to the vessel lumen via a fiber-optic based imaging catheter 826. The imaging catheter is connected through hardware to software on a host workstation. The hardware includes an imagining engine 859 and a handheld patient interface module (PIM) 839 that includes user controls. The proximal end of the imaging catheter is connected to PIM 839, which is connected to an imaging engine as shown in FIG. 3.

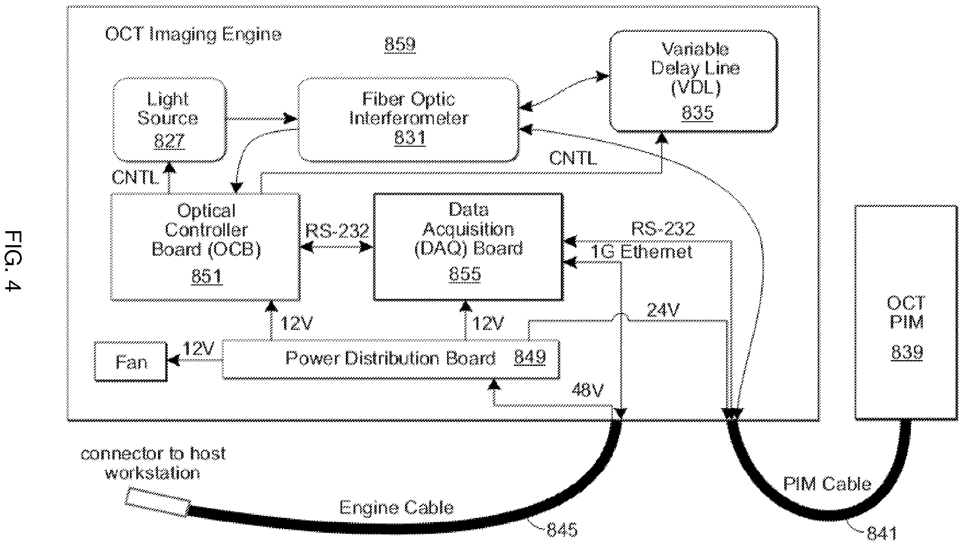

As shown in FIG. 4, the imaging engine 859 (e.g., a bedside unit) houses a power supply 849, light source 827, interferometer 831, and variable delay line 835 as well as a data acquisition (DAQ) board 855 and optical controller board (OCB) 854. A PIM cable 841 connects the imagine engine 859 to the PIM 839 and an engine cable 845 connects the imaging engine 859 to the host workstation.

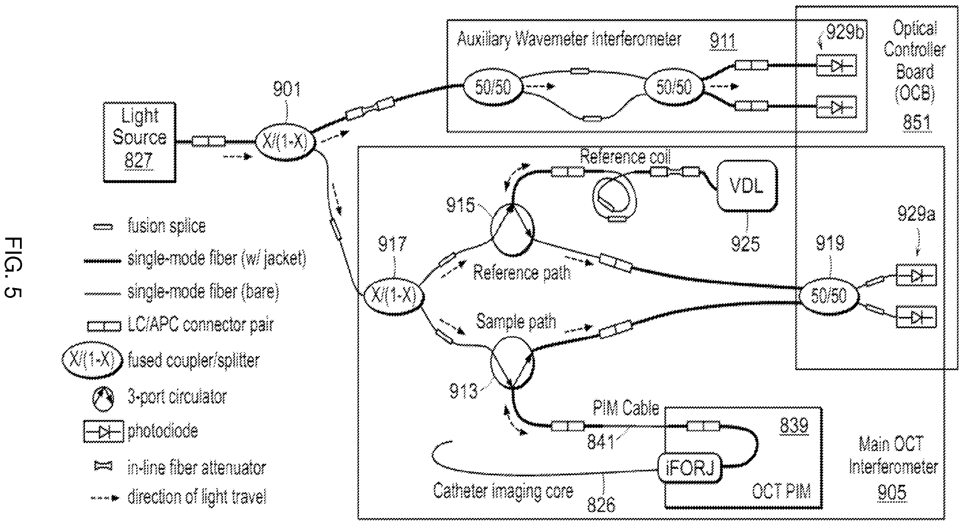

FIG. 5 shows light path in a differential beam path system according to an exemplary embodiment of the invention. Light for image capture originates within the light source 827. This light is split between an OCT interferometer 905 and an auxiliary, or "clock", interferometer 911. Light directed to the OCT interferometer is further split by splitter 917 and recombined by splitter 919 with an asymmetric split ratio. The majority of the light is guided into the sample path 913 and the remainder into a reference path 915. The sample path includes optical fibers running through the PIM 839 and the imaging catheter 826 and terminating at the distal end of the imaging catheter where the image is captured.

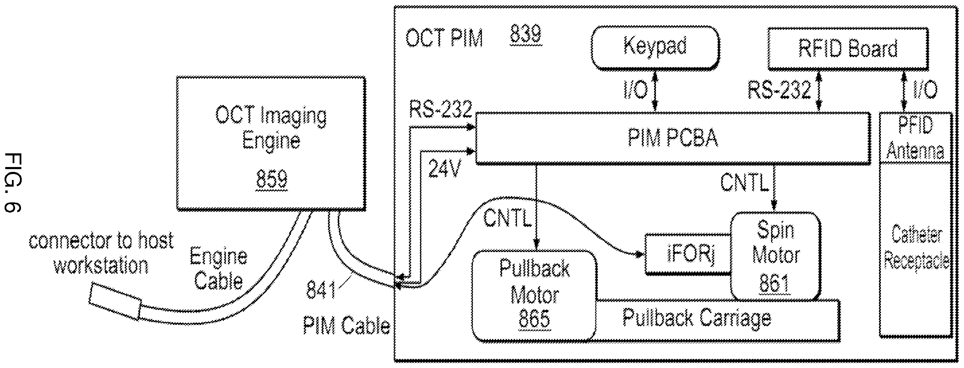

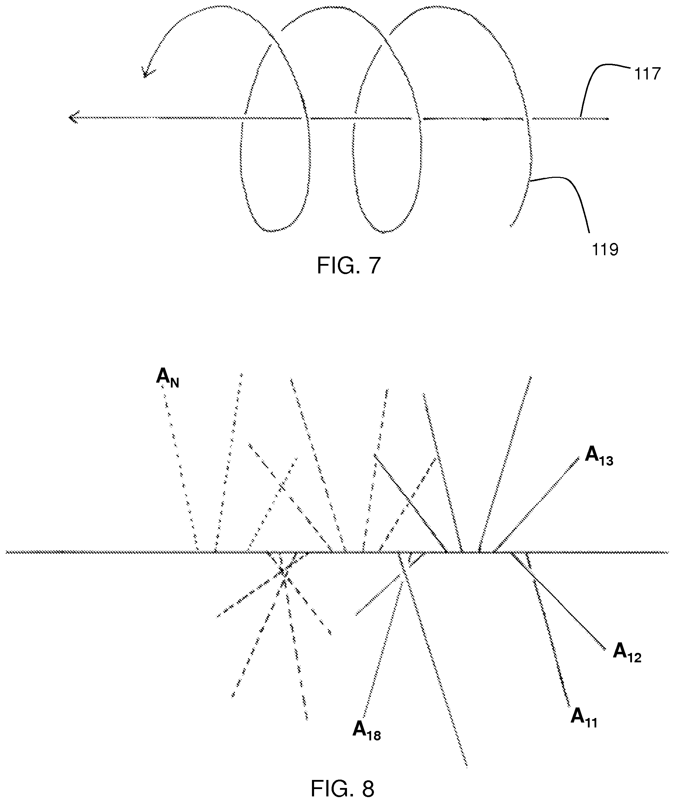

Typical intravascular OCT involves introducing the imaging catheter into a patient' s target vessel using standard interventional techniques and tools such as a guide wire, guide catheter, and angiography system. Rotation is driven by spin motor 861 while translation is driven by pullback motor 865, shown in FIG. 6. This results in a motion for image capture described by FIG. 7. Blood in the vessel is temporarily flushed with a clear solution for imaging. When operation is triggered from the PIM or control console, the imaging core of the catheter rotates while collecting image data that it delivers to the console screen. Using light provided by the imaging engine, the inner core sends light into the tissue in an array of A scan lines as illustrated in FIG. 8 and detects reflected light.

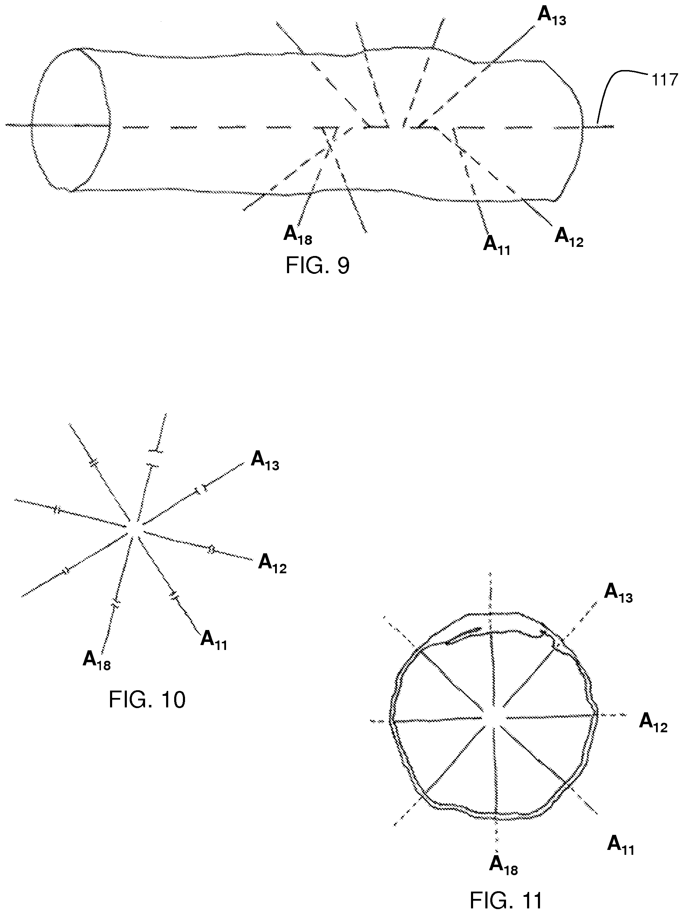

FIG. 9 shows the positioning of A scans within a vessel. Each place where one of A scans A11, A12, ..., AN intersects a surface of a feature within vessel 101 (e.g., a vessel wall) coherent light is reflected and detected. Catheter 826 translates along axis 117 being pushed or pulled by pullback motor 865.

The reflected, detected light is transmitted along sample path 913 to be recombined with the light from reference path 915 at splitter 919 (FIG. 5). A variable delay line (VDL) 925 on the reference path uses an adjustable fiber coil to match the length of reference path 915 to the length of sample path 913. The reference path length is adjusted by a stepper motor translating a mirror on a translation stage under the control of firmware or software. The free-space optical beam on the inside of the VDL 925 experiences more delay as the mirror moves away from the fixed input/output fiber.

The combined light from splitter 919 is split into orthogonal polarization states, resulting in RF-band polarization-diverse temporal interference fringe signals. The interference fringe signals are converted to photocurrents using PIN photodiodes 929a, 929b,... on the OCB 851 as shown in FIG. 5. The interfering, polarization splitting, and detection steps are done by a polarization diversity module (PDM) on the OCB. Signal from the OCB is sent to the DAQ 855, shown in FIG. 4. The DAQ includes a digital signal processing (DSP) microprocessor and a field programmable gate array (FPGA) to digitize signals and communicate with the host workstation and the PIM. The FPGA converts raw optical interference signals into meaningful OCT images. The DAQ also compresses data as necessary to reduce image transfer bandwidth to IGbps (e.g., compressing frames with a lossy compression JPEG encoder).

Data is collected from A scans A11, A 12, ..., AN and stored in a tangible, non-transitory memory. A set of A scans generally corresponding to one rotation of catheter 826 around axis 117 collectively define a B scan. FIG. 10 illustrates a set of A scans A11, A12, .. ., A18 used to compose a B scan according to certain embodiments of the invention. These A scan lines are shown as would be seen looking down axis 117 (i.e., longitudinal distance between then is not seen).

While eight A scan lines are here illustrated, typical OCT applications can include between 300 and 1,000 A scan lines to create a B scan (e.g., about 660). Reflections detected along each A scan line are associated with features within the imaged tissue. Reflected light from each A scan is combined with corresponding light that was split and sent through reference path 915 and VDL 925 and interference between these two light paths as they are recombined indicates features in the tissue.

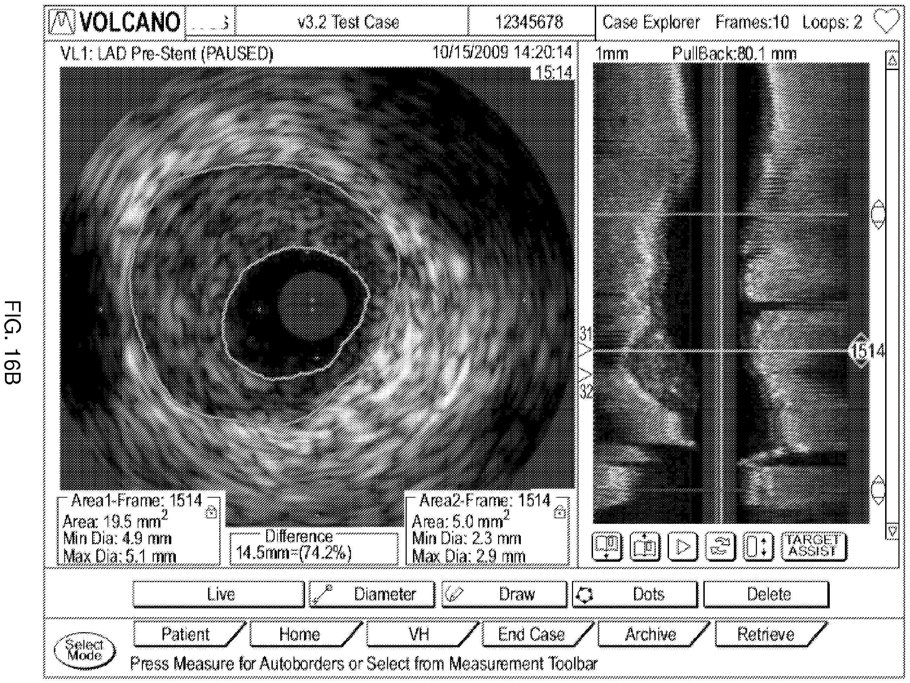

The data of all the A scan lines together represent a three-dimensional image of the tissue. The data of the A scan lines generally referred to as a B scan can be used to create an image of a cross section of the tissue, sometimes referred to as a tomographic view. For example, FIG. 11 shows the set of A scans shown in FIG. 10 within a cross section of a vessel. A B scan can be represented as a visual depiction of a cross section of a vessel (see left side of FIG. 16B).

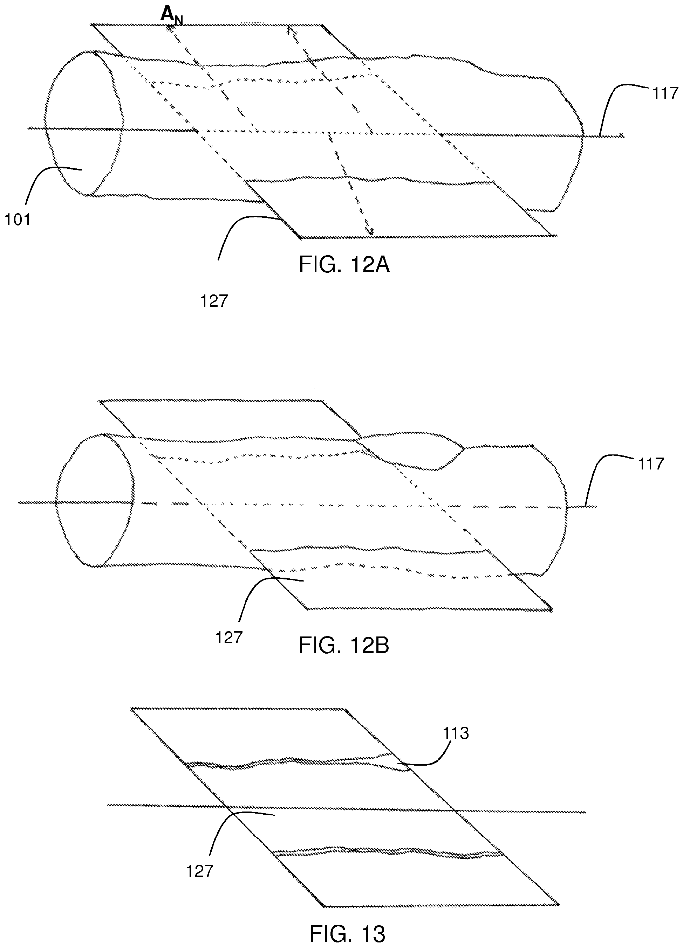

Where a B scan generally represents an image as a planar view across a vessel or other tissue (i.e., normal to axis 117), an image can also be represented as a planar view along a vessel (i.e., axis 117 lies in the plane of the view). FIG. 12A shows a longitudinal plane 127 through a vessel 101 including several A scans. Such a planar image along a vessel is sometimes referred to as an in-line digital view or image longitudinal display (ILD). As shown in FIG. 12A, plane 127 generally comprises data associated with a subset of the A scans. FIG. 12B shows a longitudinal plane through a vessel drawn without the A scan lines to assist in visualizing plane 127 comprising axis 117.

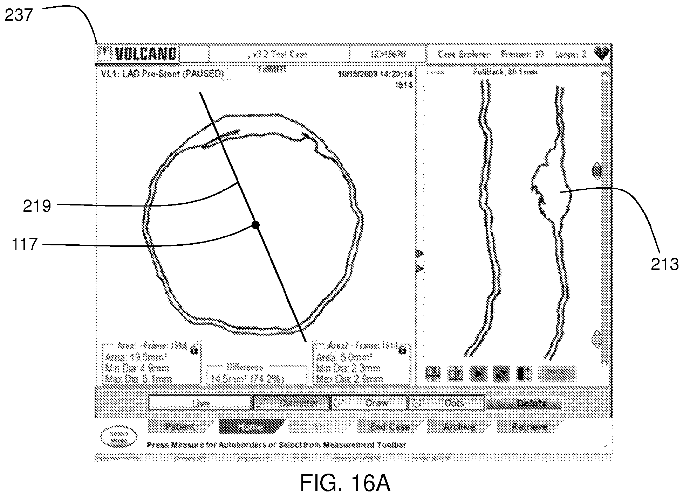

The data of the A scan lines is processed to generate images of the tissue. By processing the data appropriately (e.g., by fast Fourier transformation), a two-dimensional image can be prepared from the three dimensional data set. Systems and methods provide one or more of a tomographic view. ILD, or both. FIG. 13 is a perspective view of an idealized plane shown including an exemplary ILD in the same perspective as the longitudinal plane shown in FIGS. 12B and 12A. The ILD shown in FIG. 13 can be presented by systems and methods described herein, for example, as shown in the right area of the display illustrated in FIG. 16A.

Systems of the invention are operable with any compatible method of generating a three-dimensional image of tissue. In certain embodiments, the invention provides systems for imaging tissue using intravascular ultrasound (IVUS). IVUS uses a catheter with an ultrasound probe attached at the distal end. The proximal end of the catheter is attached to computerized ultrasound equipment. To visualize a vessel via IVUS, angiographic techniques are used and the physician positions the tip of a guide wire, usually 0.36 mm (0.014") diameter and about 200 cm long. The physician steers the guide wire from outside the body, through angiography catheters and into the blood vessel branch to be imaged.

The ultrasound catheter tip is slid in over the guide wire and positioned, again, using angiography techniques, so that the tip is at the farthest away position to be imaged. Sound waves are emitted from the catheter tip (e.g., in about a 20-40 MHz range) and the catheter also receives and conducts the return echo information out to the external computerized ultrasound equipment, which constructs and displays a real time ultrasound image of a thin section of the blood vessel currently surrounding the catheter tip, usually displayed at 30 frames/second image.

The guide wire is kept stationary and the ultrasound catheter tip is slid backwards, usually under motorized control at a pullback speed of 0.5 mm/s. Systems for IVUS are discussed in U.S. Pat. 5,771,895; U.S. Pub. 2009/0284332; U.S. Pub. 2009/0195514 A1; U.S. Pub. 2007/0232933; and U.S. Pub. 2005/0249391. Imaging tissue by IVUS produces tomographic (cross-sectional) or ILD images, for example, as illustrated in FIG. 16A and shown in FIG. 16B.

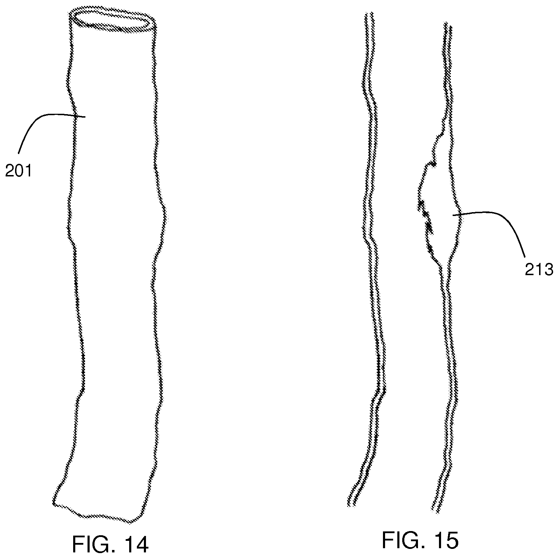

Systems of the invention allow an operator to set an image capture parameter for three dimensional imaging. In one embodiment, systems of the invention receive an image capture parameter by rendering a user interface and receiving input via an operator's use of the interface. FIG. 14 illustrates a portion of a vessel that may be imaged and FIG. 15 is a cross-sectional view of the vessel shown in FIG. 14, presented for reference in subsequent discussion. As can be seen in FIGS. 14 and 15, example target tissue 201 includes a region of interest 213. An operator may or may not have a priori knowledge of the existence of region 213.

In certain embodiments, a system for three dimensional imaging is operated to capture an image of tissue 201. An electronic apparatus within the system (e.g., PC, dedicated hardware, or firmware) such as the host workstation 433 stores the three dimensional image in a tangible, non-transitory memory and renders a display (e.g., on a screen or computer monitor) including at least a first image of tissue 201.

FIG. 16A is an illustration of a display 237 including an image of the vessel shown in FIGS. 14-15, as rendered by a system of the invention. FIG. 16B shows a display similar to that shown in FIG. 16A. The images included in display 237 in FIG. 16A are rendered in a simplified style of the purposes of ease of understanding. A system of the invention may render a display as shown in FIG. 16A, as shown in FIG. 16B, or in any style known in the art (e.g., with or without color).

In certain embodiments, display 237 is rendered within a windows-based operating system environment, such as Windows, Mac OS, or Linux or within a display or GUI of a specialized system. Display 237 can include any standard controls associated with a display (e.g., within a windowing environment) including minimize and close buttons, scroll bars, menus, and window resizing controls (not shown in FIGS. 16-19). Elements of display 237 can be provided by an operating system, windows environment, application programing interface (API), web browser, program, or combination thereof (for example, in some embodiments a computer includes an operating system in which an independent program such as a web browser runs and the independent program supplies one or more of an API to render elements of a GUI). Display 237 can further include any controls or information related to viewing images (e.g., zoom, color controls, brightness/contrast) or handling files comprising three-dimensional image data (e.g., open, save, close, select, cut, delete, etc.). Further, display 237 can include controls (e.g., buttons, sliders, tabs, switches) related to operating a three dimensional image capture system (e.g., go, stop, pause, power up, power down).

In certain embodiments, display 237 includes controls related to three dimensional imaging systems that are operable with different imaging modalities. For example, display 237 generally may include start, stop, zoom, save, etc., buttons, and be rendered by a computer program that interoperates with OCT or IVUS modalities. Thus display 237 can display an image to a user derived from a three-dimensional data set with or without regard to the imaging mode of the system.

Display 237 includes an image of tissue 201. As shown in FIG. 16A, display 237 includes two images of tissue 201, a tomographic view and an ILD. Display 237 can include indicia to show a relationship between the content of the ILD and the tomographic view such as, for example, a line 219 across the tomographic view comprising axis 117 and showing the section of tissue 201 that the ILD represents.

Systems of the invention are configured to receive input from an operator that comprises a selection of a portion of an image in display 237. An operator may select part of an image in display 237 by any method known in the art including dragging a mouse pointer over a portion of the display, touching a touch-sensitive screen, clicking a button to confirm a proposed selection (for example, as automatically generated by a computer program), or through interacting with one or more markers presented in display 237.

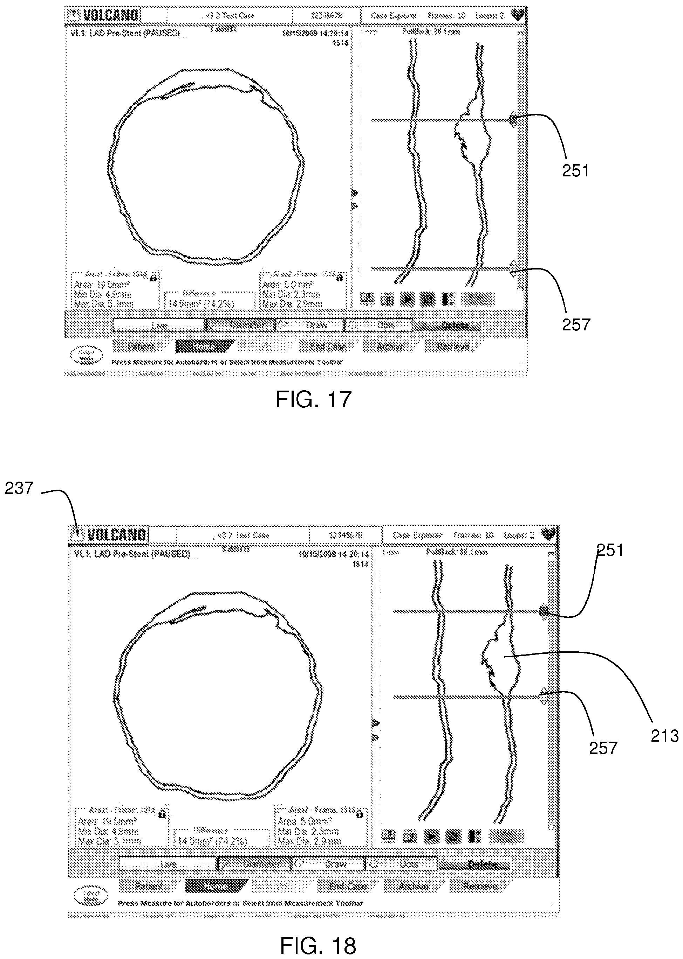

FIG. 17 is an illustration of a display including parameter-setting markers shown on the ILD. Start marker 251 and end marker 257 can be rendered by an electronic computing device within display 237. These markers can be color-coded (e.g., green for start and red for end), animated (e.g., "marching ants" dotted line), transient (e.g., only appear when mouse pointer is hovered near certain portion of screen, or have any other quality associated with elements in a GUI). Markers can be used to mark a portion of the display and can be positioned on display 237 via an operator's interaction with a computer system (e.g., host workstation 433) including, for example, by dragging with a mouse, use of arrow keys, dragging on a touch screen or touch pad, typing in numbers, or using auto-find commands proffered by imaging software.

FIG. 18 is an illustration of the display shown in FIG. 17 in which the markers have been positioned to represent a selection of points within the ILD in the display. Start marker 251 and end marker 257 generally establish a boundary defining a region of interest 213. In some embodiments, start marker 251 and end marker 257 provide tools for measuring an actual dimension of region 213. For example, where region 213 represents an adverse thrombotic feature, markers on an ILD in display 237 can be used to measure its length along vessel 201. Similar markers on a tomographic view (not shown) can be used to measure a circumferential distance of region 213. Further, similarly, markers (or mouse drag operations) can be used to measure a thickness of a feature within tissue. Since an image capture system of the invention presents display 237 based on a three dimensional data file, systems of the invention can use a position of markers on a screen to calculate a dimension in three-dimensional space of the target tissue being imaged.

Systems of the invention utilize a selection to establish a boundary defining a region of tissue 201. In certain embodiments, the boundary is established by a processor in an electronic device such as host workstation 433. For example, where an operator positions markers in a display at boundaries of an image of a region 213, systems of the invention can establish a corresponding boundary in three-dimensional space of the target tissue being imaged. This boundary can be calculated by a processor and stored, for example as a set of coordinates, in a tangible, non-transitory memory.

Using a boundary established based on a received selection, an image can be captured that includes region 213 and no surrounding portion of tissue 201. In certain embodiments, one image capture event, or "pullback", of an imaging system captures a fixed amount of data and imaging a smaller total area of tissue thus produces a higher level of detail, or resolution. Resolution as used herein does not strictly necessarily refer to dots or pixels per unit measurement (although that is one included exemplary definition) and is not limited to digital data but rather encompasses digital data as well as non-digital data such as light-based image data (e.g., stored on film). Resolution here refers to a level of detail or a smallest dimension of a feature that can be perceived and understood via a system or display. For a fixed quantum of input data, capturing an image of a smaller portion of tissue offers a higher resolution than capturing an image of a larger portion of the tissue. Systems of the invention capture a second image of the tissue within a designated boundary established by receiving a user selection. The user selection is made by interacting with a first image, for example, within display 237 or a GUI using techniques described herein.

In certain embodiments, an imaging system captures both the first and the second image, for example, in a single procedure. Catheter 826 is inserted into vessel 201, e.g., as described above. An operator triggers operation via PIM 839 or via a host workstation, for example, through use of a button or menu in a GUI. A three-dimensional image is captured and stored in a tangible, non-transitory medium and imaging engine 859 provides data that is rendered into a tomographic view and an ILD as shown in FIG. 17. An operator positions markers 251 and 257 at boundaries of region of interest 213 and triggers a second image capture operation. A second image is captured including only region 213 and having a higher resolution than the first image.

In certain embodiments, one or more operations or steps of an operation are performed automatically by devices or systems. Automatically generally describes an operation or step that occurs without human intervention between it and some related or causal step or operation. In certain embodiments, a boundary corresponding to a selected portion of an image (selected points) is established, a three dimensional image is captured, or both, automatically. For example, systems and the invention can operate automatically and responsive to any step of operator input (e.g., a mouse release, a key stroke, a lapse of time without an input) to trigger an operation or step.

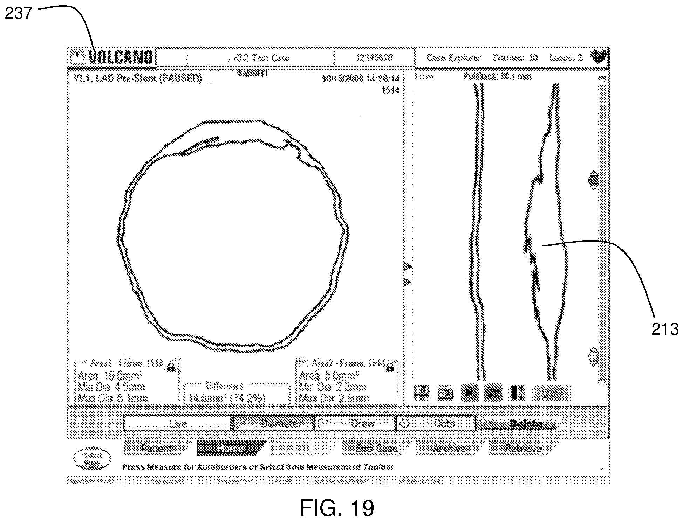

FIG. 19 is an illustration of a display including a second image of the portion of the vessel corresponding to the selection represented by the position of the markers shown in FIG. 18. Here, region 213 occupies the entire ILD due to the operator's positioning of start marker 251 and end marker 257 (as seen in FIG. 18). This second ILD image and the corresponding second tomographic image (left side of display 237 in FIG. 19) are generated from the three dimensional data set generated by the imaging operation. Due to the fact that the tomographic view and the ILD each correspond to the same three dimensional target, are two dimensional views, and each represent different dimensions than the other, display 237 is said to include a three dimensional image. Furthermore, the data set from which display 237 is generated represents information in three dimensions about tissue 201.

In certain embodiments, systems of the invention render a GUI with elements or controls to allow an operator to interact with three dimensional data set as a three dimensional view. For example, an operator may cause a video affect to be viewed in, for example, a tomographic view, creating a visual effect of travelling through a lumen of vessel 201 (i.e., a dynamic progress view). Noting that a dynamic progress view (e.g., video) representing travel through a lumen of vessel 201 corresponds to a progression in a vertical direction along an ILD as shown , for example, in FIG. 17, an operator may select points from within one of the images or the three dimensional data set by choosing start and stop points while a dynamic progress view is displayed in display 237 (e.g., interact with tomographic view to choose points from within ILD).

In certain embodiments, an operator chooses a start and stop point by interacting with a tomographic view using a computer device (e.g., host workstation 433) while a dynamic progress view plays, for example, by tapping space bar for start and space bar for stop, or by clicking on the display with a mouse at moments in the dynamic progress view corresponding to start and stop points. In certain embodiments, holding down a key (e.g., "up arrow" key) causes a dynamic progress view with a "forward motion" effect and holding down another key (e.g., "down arrow") causes a reverse motion effect. Systems of the invention can thus receive a selection of points within the first image (e.g., through interaction with the tomographic image) and optionally display start marker 251 and end marker 257 on the ILD in positions corresponding to the operator's interactions.

Certain imaging systems such as some existing OCT systems have a default 10 cm pullback length and produce images in which a default ILD represents a 10 cm length of vessel 201. Where a user selects points from within the first image corresponding to 25% of an ILD, an imaging system will then perform an image capture operation with a 2.5 cm pullback capturing an image of the target tissue as indicated by the selection. Here, in this example, the second image will have a resolution of 4X that of the first image.

While generally described here with reference to start marker 251 and end marker 257, a user may provide a selection of points within an image by interacting with any visual queue, element of a GUI, or hardware input. For example, a user may trigger operation of a plug-in or application that analyzes a first image and automatically detects an anomaly or feature and generates a selection based on the automatic detection. A user may operate an imaging system in such a way as to generate a selection based on a physical or biological phenomenon exhibited on a component of the system. For example, where pullback motor 865 generates a constant torque during a pullback, in some embodiments a system is programmed to select a region of the pullback in which the catheter travels the slowest, i.e., was subject to relatively high drag forces within the target (e.g., measured by current draw at pullback motor 865).

In certain embodiments, a user employs a macro to cause a recursive or iterative image capture operation. A macro generally refers to an operation routine including a number of steps preferably programmed to run automatically once triggered. For example, a user may designate a single point within an ILD and cause the system to capture the 10% of the ILD surrounding the point, display the high resolution second image, capture the 10% of the second ILD surrounding the same point, display the higher still resolution third image, and so on, for any number of cycles (recursive model). In an iterative model, a user my select a region of a first image (e.g., 10% of the ILD) and cause a system to perform one image capture operation of the first 10% of the selection (i.e., 1% of the first ILD), one image capture operation of the second 10% of the selection,...and so on, until the first selected region has been imaged by 10 pullbacks creating a final image with 100X resolution compared to the first image.

In certain embodiments, start marker 251 and end marker 257 operate as rulers to measure a dimension or to control video playback while also operating as a mechanism by which an operator inputs a selection. Thus, an operator may examine a first image in dynamic progress view or in any other video-type playback mode and use the markers to establish parameters of the video. The operator may then confirm to the system that the markers also represent the selection to be used to establish a boundary for a subsequent image capture operation. Note that this process can proceed iteratively. An operator can view the second image in dynamic progress view, for example, and again choose a target region for a third imaging operation, and so on.

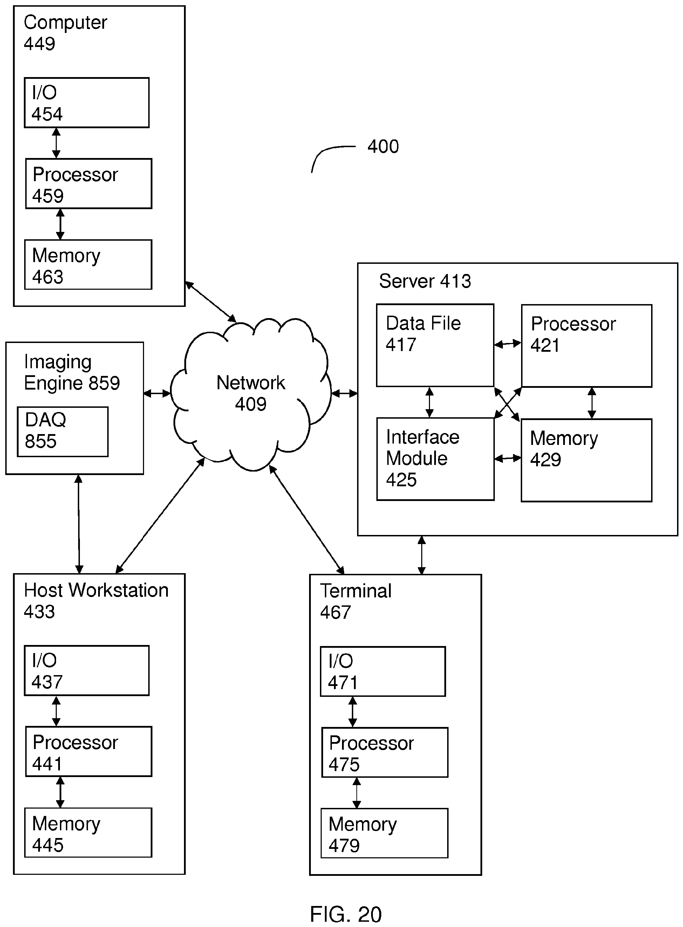

In some embodiments, a user interacts with a visual interface and puts in parameters or makes a selection. Input from a user (e.g., parameters or a selection) are received by a processor in an electronic device such as, for example, host workstation 433, server 413, or computer 449. The selection can be rendered into a visible display. An exemplary system including an electronic device is illustrated in FIG. 20. As shown in FIG. 20, imaging engine 859 communicates with host workstation 433 as well as optionally server 413 over network 409. In some embodiments, an operator uses host workstation 433, computer 449, or terminal 467 to control system 400 or to receive images. An image may be displayed using an I/O 454, 437, or 471, which may include a monitor. Any I/O may include a keyboard, mouse or touchscreen to communicate with any of processor 421, 459, 441, or 475, for example, to cause data to be stored in any tangible, nontransitory memory 463, 445, 479, or 429. Server 413 generally includes an interface module 425 to effectuate communication over network 409 or write data to data file 417. Methods of the examples can be performed using software, hardware, firmware, hardwiring, or combinations of any of these. Features implementing functions can also be physically located at various positions, including being distributed such that portions of functions are implemented at different physical locations (e.g., imaging apparatus in one room and host workstation in another, or in separate buildings, for example, with wireless or wired connections). In certain embodiments, host workstation 433 and imaging engine 855 are included in a bedside console unit to operate system 400.

Processors suitable for the execution of computer program include, by way of example, both general and special purpose microprocessors, and any one or more processor of any kind of digital computer. Generally, a processor will receive instructions and data from a read-only memory or a random access memory or both. The essential elements of computer are a processor for executing instructions and one or more memory devices for storing instructions and data. Generally, a computer will also include, or be operatively coupled to receive data from or transfer data to, or both, one or more mass storage devices for storing data, e.g., magnetic, magneto-optical disks, or optical disks. Information carriers suitable for embodying computer program instructions and data include all forms of non-volatile memory, including by way of example semiconductor memory devices, (e.g., EPROM, EEPROM, NAND-based flash memory, solid state drive (SSD), and other flash memory devices); magnetic disks, (e.g., internal hard disks or removable disks); magneto-optical disks; and optical disks (e.g., CD and DVD disks). The processor and the memory can be supplemented by, or incorporated in, special purpose logic circuitry.

To provide for interaction with a user, the subject matter described herein can be implemented on a computer having an I/O device, e.g., a CRT, LCD, LED, or projection device for displaying information to the user and an input or output device such as a keyboard and a pointing device, (e.g., a mouse or a trackball), by which the user can provide input to the computer. Other kinds of devices can be used to provide for interaction with a user as well. For example, feedback provided to the user can be any form of sensory feedback, (e.g., visual feedback, auditory feedback, or tactile feedback), and input from the user can be received in any form, including acoustic, speech, or tactile input.

The subject matter described herein can be implemented in a computing system that includes a back-end component (e.g., a data server 413), a middleware component (e.g., an application server), or a front-end component (e.g., a client computer 449 having a graphical user interface 454 or a web browser through which a user can interact with an implementation of the subject matter described herein), or any combination of such back-end, middleware, and front-end components. The components of the system can be interconnected through network 409 by any form or medium of digital data communication, e.g., a communication network. Examples of communication networks include cell networks (3G, 4G), a local area network (LAN), and a wide area network (WAN), e.g., the Internet.

The subject matter described herein can be implemented as one or more computer program products, such as one or more computer programs tangibly embodied in an information carrier (e.g., in a non-transitory computer-readable medium) for execution by, or to control the operation of, data processing apparatus (e.g., a programmable processor, a computer, or multiple computers). A computer program (also known as a program, software, software application, app, macro, or code) can be written in any form of programming language, including compiled or interpreted languages (e.g., C, C++, Perl), and it can be deployed in any form, including as a stand-alone program or as a module, component, subroutine, or other unit suitable for use in a computing environment. Systems of the invention can include programming language known in the art, including, without limitation, C, C++, Perl, Java, ActiveX, HTML5, Visual Basic, or JavaScript.

A computer program does not necessarily correspond to a file. A program can be stored in a portion of file 417 that holds other programs or data, in a single file dedicated to the program in question, or in multiple coordinated files (e.g., files that store one or more modules, subprograms, or portions of code). A computer program can be deployed to be executed on one computer or on multiple computers at one site or distributed across multiple sites and interconnected by a communication network.

A file can be a digital file, for example, stored on a hard drive, SSD, CD, or other tangible, non-transitory medium. A file can be sent from one device to another over network 409 (e.g., as packets being sent from a server to a client, for example, through a Network Interface Card, modem, wireless card, or similar).

Writing a file according to the invention involves transforming a tangible, non-transitory computer-readable medium, for example, by adding, removing, or rearranging particles (e.g., with a net charge or dipole moment) into patterns of magnetization by read/write heads, the patterns then representing new collocations of information desired by, and useful to, the user. In some embodiments, writing involves a physical transformation of material in tangible, non-transitory computer readable media with certain properties so that optical read/write devices can then read the new and useful collocation of information (e.g., burning a CD-ROM). In some embodiments, writing a file includes using flash memory such as NAND flash memory and storing information in an array of memory cells include floating-gate transistors. Methods of writing a file are well-known in the art and, for example, can be invoked automatically by a program or by a save command from software or a write command from a programming language.

An example provides an automatic longitudinal image playback system and method for three dimensional medical imaging. Systems and methods of the example receive a three dimensional data set and display a series of coaxial longitudinal images (i.e., each rotationally offset from another around an axis) in sequence, creating a video effect as if the view were rotating through the tissue. Since the video view plays without simultaneous hands-on operation by a user, a user is free to operate the image capture controls of the system while visually inspecting the subject tissue in three dimensions through the display. Where the tissue includes a feature of interest, the user may establish scan parameters such as a start or stop point while seeing the three dimensional shape, orientation, and extent of the feature in the display. This allows the user to make a high resolution close-up scan that is directed at the feature accurately and with precision.

Display 237 includes an image of tissue 201. As shown in FIG. 16A, display 237 includes two images of tissue 201, a tomographic view and a longitudinal view, as well as a longitudinal marker 219 across axis 117. In a display according to FIG. 16A, longitudinal marker 219 represents the present cross-section shown in the longitudinal view on the right portion of the display. The example provides a longitudinal video playback mode. While a video according to the example is being played, longitudinal marker 219 can be shown rotating around axis 117 as the longitudinal view on the right side of the screen changes correspondingly. In certain examples, the example provides a display 237 that automatically plays a video of the longitudinal display or includes tools to cause such a video to be played.

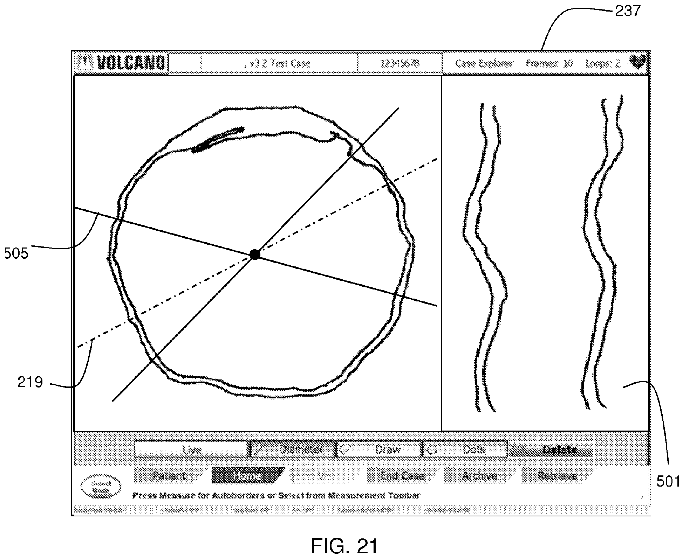

Systems of the example are configured to receive input from an operator that comprises a selection of a portion of an image in display 237. FIG. 21 is an illustration of a display including selection tools. An operator may select part of an image in display 237 by any method known in the art including dragging a mouse pointer over a portion of the display, touching a touch-sensitive screen, clicking a button to confirm a proposed selection (for example, as automatically generated by a computer program), keying in positional data, or through interacting with one or more markers presented in display 237. In certain embodiments, an operator positions one or more of video marker 505 as shown in FIG. 21 to select a portion of vessel 201 to be shown in longitudinal video view 501. A marker 505 can be positioned on screen by any suitable method known in the art including, for example, dragging with a mouse, typing in a value (e.g., of degrees offset from an origin such as an idealized horizontal line), or confirming a selection made automatically such as by a program or plug in that analyzes data in the three dimensional image set.

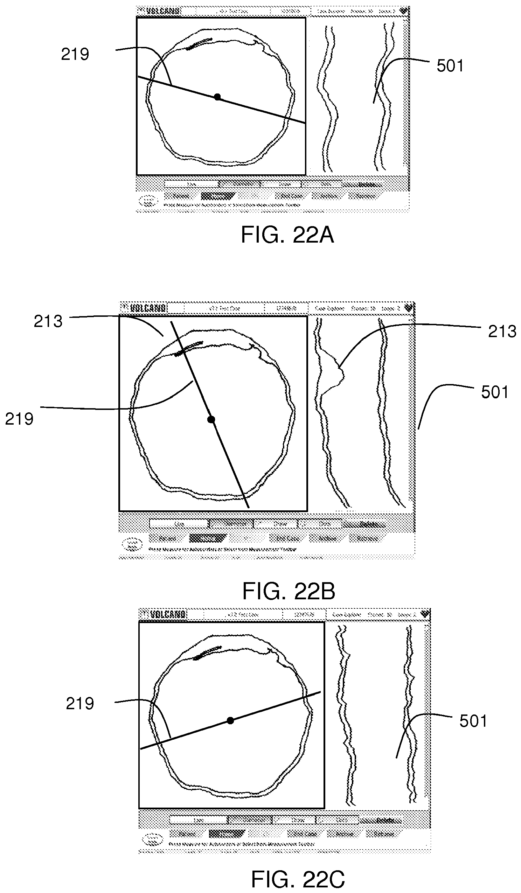

FIGS. 22A-22C show a series of displays that present a video. As can be understood by visualizing FIGS. 22A-22C sequentially, as longitudinal marker 219 rotates, corresponding longitudinal video view 501 progressively shows different, corresponding portions of tissue 201 (Note that in FIG. 22B, as compared to FIG. 22A or FIG. 22B, longitudinal marker 219 transects feature 213 and that feature 213 is only visible in a longitudinal view in FIG. 22B).

In certain embodiments, the example provides a window in a GUI that displays a video. For example, where display 237 is presented in a window of a GUI, a video can be viewed within display 237 or can be shown in a new window that opens to display the video, optionally with controls specific to playing video.

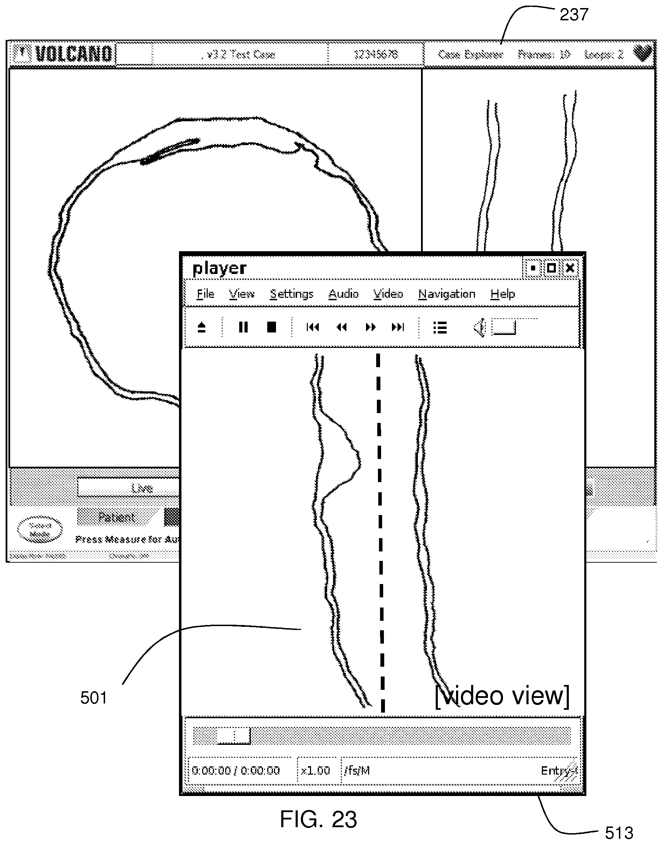

FIG. 23 is a two-window display according to an example. As shown in FIG. 23, video player 513 is presented as a window that is dedicated to showing and controlling videos. Video player 513 can be invoked automatically responsive to a predetermined condition, such as capturing a three dimensional image by scanning with a system, or player 513 can be invoked by command of a user.

In some examples player 513 is not rendered as an independent window, but instead "takes over" display 237 for the purposes of playing longitudinal video 501.

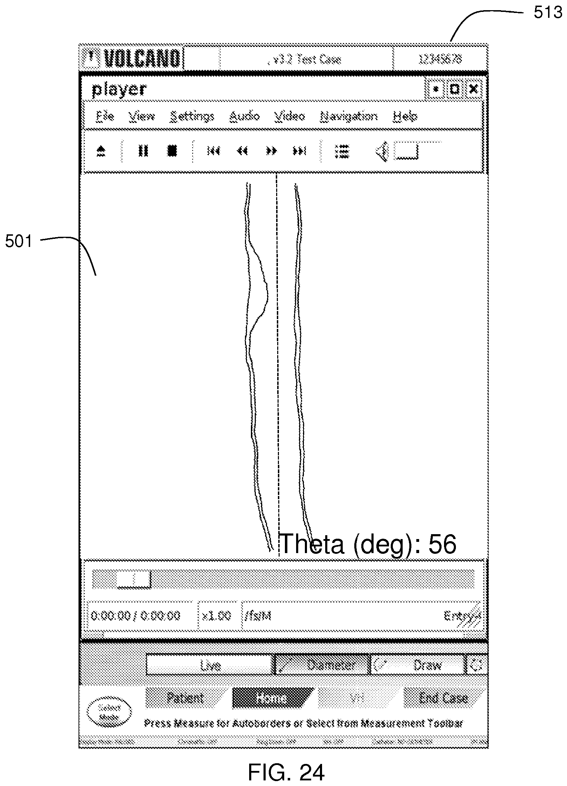

FIG. 24 is a video player display according to an example using a substantial number of the GUI elements presented in display 237. As shown in FIG. 24, the visible position of the ILD is indicated by a text label showing an offset in degrees (here, 56 degrees) from an origin (e.g., an arbitrary origin, or the orientation of the first A scan line).

As shown in any example or as can be shown in any equivalent, a video longitudinal view 501 can be presented to the operator of a system. An operator can establish parameters to control playback of the video, such as start and stop position, number of times to loop, duration to play for (e.g., in min, sec, or both), speed, direction, etc. Controls can be presented to the operator in any format. For example, in some embodiments, display 237 includes controls such as a play button (e.g., a green triangle). In certain examples, when an operator clicks on a play button (for example, with a computer pointing device like a mouse or by touching a touch screen), and ILD video is presented in loop mode-playing through 360 degrees of ILD data, repeatedly until the operator stops playback (e.g., by clicking on a red square stop button). A user may be given the option for the system to always begin automatic playback after a pullback of an OCT or IVUS system is complete.

A user may optionally control what portion of a series of ILDs is presented in a video. FIGS. 22-25 generally illustrate a video in which an entire ILD (i.e., with axis 117 in the center) is shown in a video. A video can also be made including only a selected area (e.g., a user-specified rectangle at user- specified dimension and position relative to axis 117) of an ILD. In this way, the view presented in longitudinal video view 501 could be said to "revolve" around axis 117 instead of rotate (axis 117 is outside of the view).

Examples further provides elements via a GUI that can be overlaid onto the longitudinal video view 501 for further functionality. For example, a user may position markers or draw a box with a mouse and the display may provide corresponding measurements of the tissue. An operator may "tag" a portion of a video, such as a portion of an image, and label the portion, for example, with a text image. Thus, when an operator spots a feature of interest (such as a biological feature like a plaque, a medical feature such as a stent, or a system related feature such as a guide wire), a user can tag the feature (e.g., drag the mouse to draw a box around the area) and add a tag, such as a text label. As discussed below, a video may be saved for sending or later viewing. When a feature is tagged, the tagged information is preserved with the saved file and can later be accessed, modified, or viewed. For example, where a plaque or a stent is labeled as such, text can be entered ("plaque AS2023"). If the video is later viewed with a compatible viewer, or is exported and saved with a "text tags visible" setting, the text will then be seen as a label of the feature in question.

While a set of ILDs generally represents 360 degrees around an axis, the ILD angles may not be evenly spaced, nor must they represent a full 360 degree view. In certain examples, an ILD set is saved by default that represents a 180 degree view, in view of the fact that each ILD is, by definition, a mirror image of the ILD that represents a 180 degree rotation of it. Thus, the ILD at 190 degrees from origin can be shown by using the ILD at 10 degrees from origin, flipped. By exploiting this redundancy, the computational power and memory required for video processing, playing, exporting or saving can be reduced.

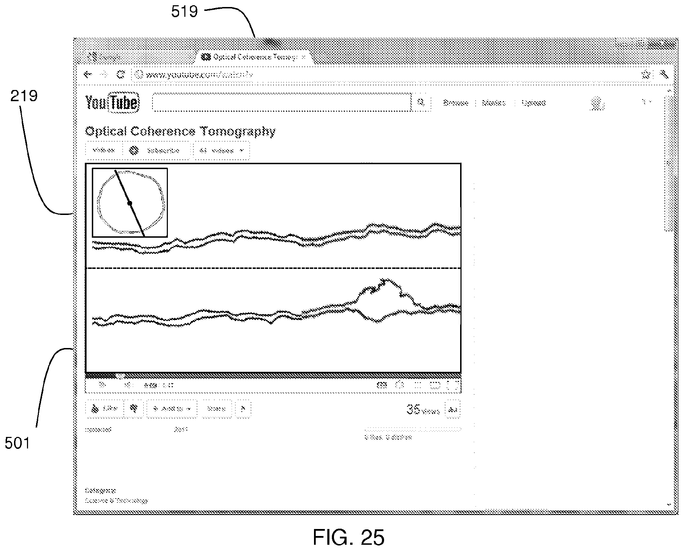

In certain examples, a longitudinal video view 501 can be saved as a file (e.g., written to a tangible, non-transitory memory) or shared, for example, using the internet. FIG. 25 shows a video interface in a web browser. As shown in FIG. 25, longitudinal video view 501 includes longitudinal marker 219. The views shown in longitudinal video view 501 are prepared from a three dimensional data set representing tissue and captured by a medical imaging system. The primary longitudinal view is a set of coaxial longitudinal views each rotationally offset from the other around an axis corresponding to an axis of translation of an imaging catheter of the imaging system. The video has been saved and made available for viewing via the internet (for example, as shown here, viewable through a web browser), thus enabling a physician or research scientist to effectively communicate medically significant findings.

In some examples, a user interacts with a visual interface and puts in parameters or makes a selection related to defining, controlling, or presenting a longitudinal video view. Input from a user (e.g., parameters or a selection) are received by a processor in an electronic device. The selection can be rendered into a visible display. An exemplary system including an electronic device is illustrated in FIG. 20.

A further example generally relates to the removal of guidewire artifacts in medical images obtained from tomographic imaging systems that use a rotational imaging probe in parallel with a guidewire. As the imaging probe rotates to obtain an image, the guidewire often appears as an artifact within the image data set and resulting images. Through use of image processing techniques, artifacts are removed from the imaging data set in order to improve image quality and interpretability.

In certain examples, in order to remove a guidewire artifact or other shadowing from an image, at least two images are taken of an imaging surface having the guidewire or other shadow causing obstruction moved or rotated to a different location. The resulting images are of the same imaging surface region having the resulting guidewire artifact or shadow in a different location. The guidewire artifact or shadow within one of the images is then replaced with data representing the imaging surface at the same location from one of the other images. This substitution removes the guidewire artifact from the image and provides a true substitution of the data representing the imaging surface lost to the shadow.

Accordingly, although aspects of the invention are generally applicable to rotational imaging systems, this invention is also contemplated for use in any medical imaging system that has a constant artifact or shadow within the imaging region that can be relocated, so that in image post-processing the artifact within one image can be replaced with data representing the imaging surface from another image. Such medical imaging systems include, for example, optical coherence tomography systems, intravascular ultrasound ("IVUS") systems, spectroscopy, photo-acoustic tomography systems, combined ultrasound and OCT systems, RAMAN, alternative interferometric techniques, computed tomography, and any other tomographic imaging technique. Although the exemplifications described herein are drawn to the invention as applied to an OCT system, at least all of these techniques are contemplated for use with the systems of the present invention. IVUS and OCT are discussed in detail above.

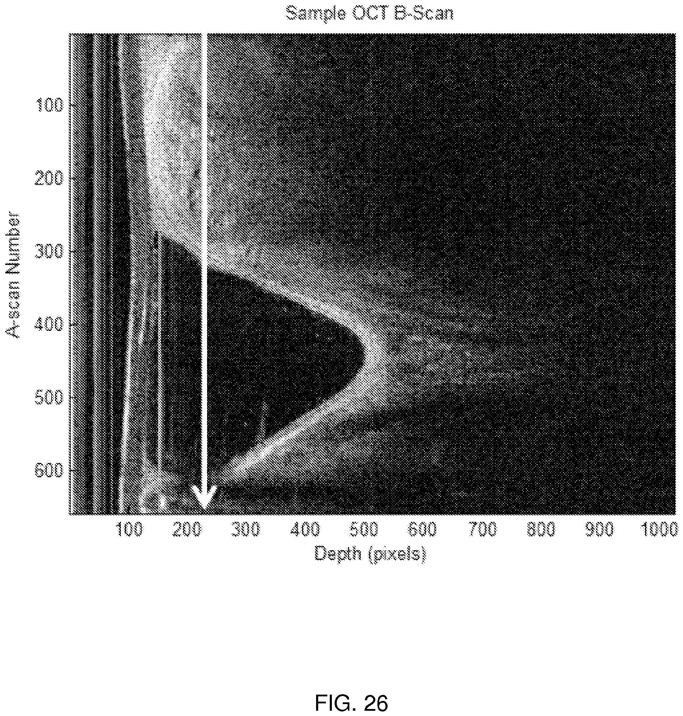

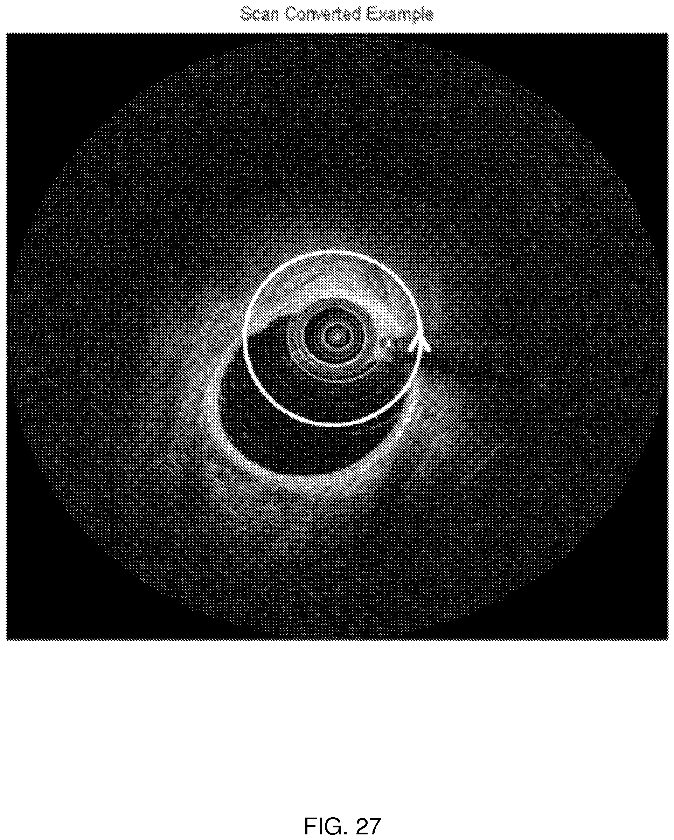

FIG. 26 is an example of an OCT polar coordinate B-Scan with 660 A-scans. To create a final tomographic view of the vessel, the B-scan is scan converted to a Cartesian coordinate system. FIG. 27 displays the scan converted image of the B-scan in FIG. 26.

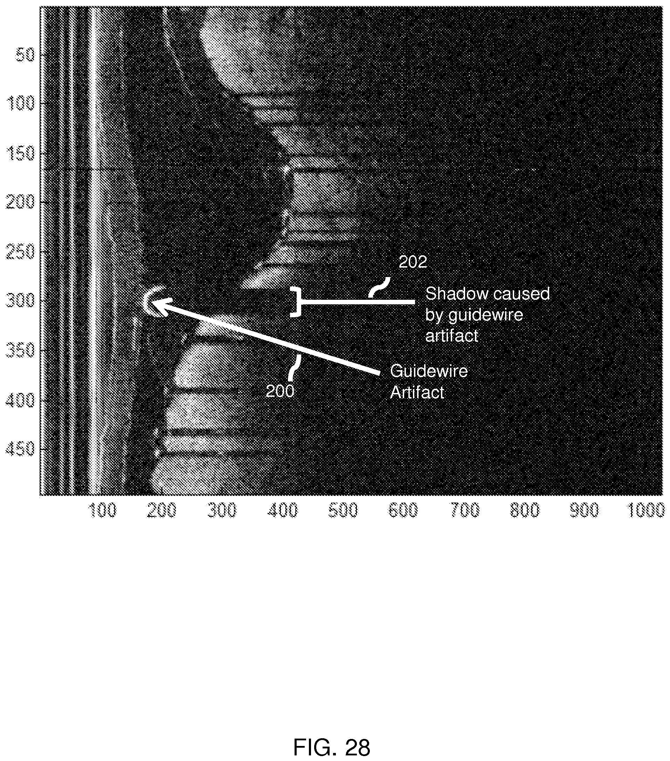

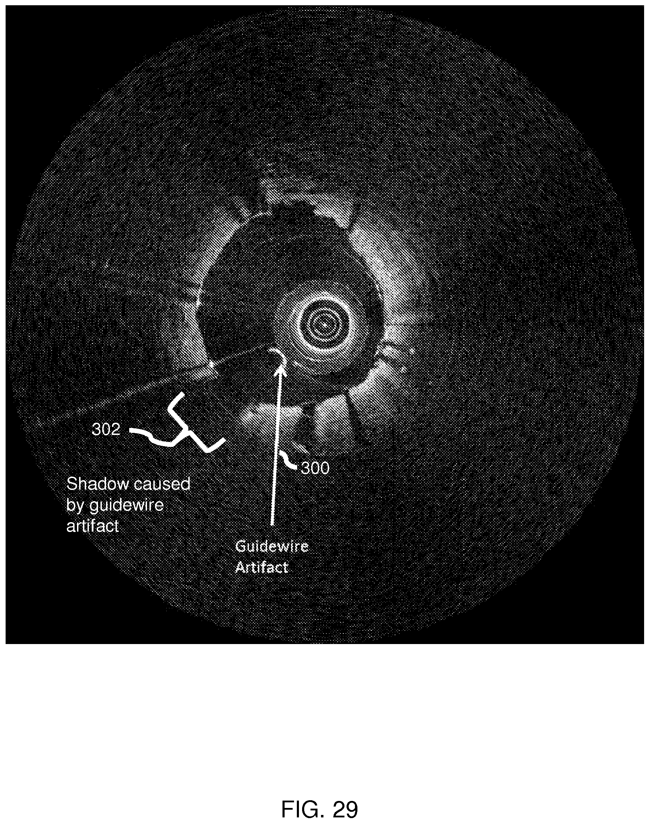

During acquisition of imaging data of the luminal surface that forms the A-scans, data can be lost due to the obstruction of a guidewire, or multiple guidewires. FIG. 28 depicts a B-scan image having a guidewire artifact 200 identified. Because the guidewire artifact 200 casts a shadow 202 outwardly (in a radially increasing direction) on a portion of the luminal surface and any features that would otherwise be visible on or beneath the luminal surface are obscured. FIG. 29 depicts a scan-converted image of a B-scan highlighting a guidewire artifact 300 and shadow 302 caused by the guidewire artifact 300.

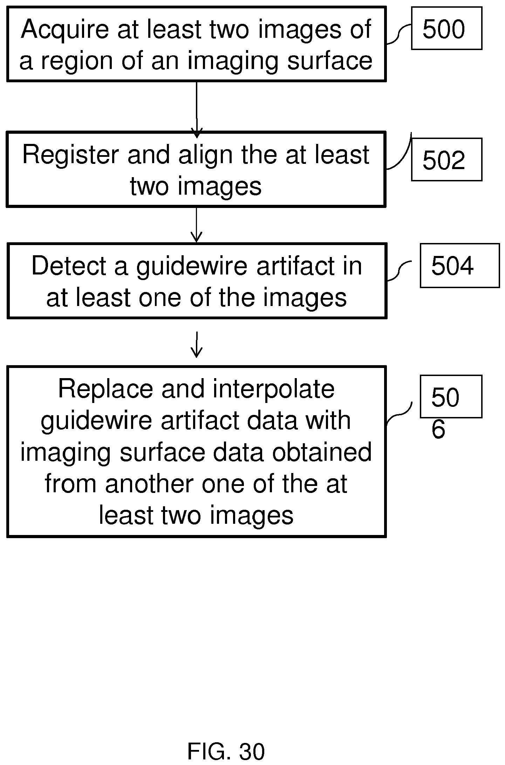

FIG. 30 exemplifies the steps employed in an example for removing guidewire artifacts. In step 500, at least two images are obtained of an imaging surface, in which each image is formed form an imaging data set. In step 502, the images are registered and aligned. In step 504, the guidewire artifact is detected in at least one of the images. In step 506, the data representing the guidewire artifact is replaced with data representing the imaging surface obtained from at least one other image.

In one example at least two images are obtained of the same imaging surface by repeating imaging data acquisition. For example, repeating imaging pull-backs or push-forwards of the imaging catheter to obtain the desired amount of images of the same imaging region. For each repeated data acquisition of the imaging region, the guidewire is moved or rotated to different position. It should be noted that the at least two images of the imaging surface are not limited to images acquired from one imaging system, rather images can be acquired from two or more different imaging systems. For example, one data set can be obtained from an OCT imaging catheter and another dataset can be obtained from an ultrasound imaging catheter. In such examples, the data representing the guidewire artifact in an OCT image is replaced with data representing the luminal surface at the same location in the ultrasound image.

In step 502, the at least two images are registered and aligned to obtain the Cartesian coordinates, XYZ coordinates, of all frames and pixels in the imaging data set for each image. Image registration is the process of determining the correspondence between all points in two images of the same scene. With respect to removing the guidewire artifact, aligning the data sets allows for one to compare the position of a guidewire artifact within one imaging data set with the imaging surface at the same position in another imaging data set. This step can occur before or after step 504 without affecting the outcome of the method. Exemplary techniques for registering images are described in, for example, Joseph Hajnal, Derek Hill, and David Hawkes, editors. Medical Image Registration. CRC Press, 2001, D. I. Barnea and H. F. Silverman, "A class of algorithms for fast digital image registration", IEEE Trans. Computers, vol. 21, no. 2, pp. 179-186, 1972. For imaging data sets obtained from more than one imaging system, an exemplary technique for registering such multimodal images is described in Acharya, et al. "Image registration of multimodality 3-D medical images by chamfer matching" Proc. SPIE 1660, 356 (1992).

In one example, the images are registered using a phase-correlation technique. Generally, a phase-correlation technique takes two input images to produce a third imaging data set, or image, which contains a single peak. The location of this peak corresponds to the relative translation between the images. The phase correlation method is resilient to noise, occlusions, and other defects typical of medical images. Additionally, the phase-correlation uses a fast Fourier transform to compute the cross-correlation between the two images, which generally results in large performance gains. Exemplary phase-correlation registration techniques are described in Givens et al, U.S. Patent No. 5,581,638, Dong et al, U.S. Patent No. 6,373,970, and Reddy et al., IEEE Transaction on Image Processing, Vol. 5, No. 8 August 1996.

Step 504 involves detecting a guidewire artifact within at least one of the images. Preferably, the guidewire artifact is detected in at least two of the acquired images. This allows one to determine whether the guidewire artifact is in a different location across datasets, and prevents the inadvertent substitution of data representing guidewire artifact with data representing guidewire artifact. Detecting guidewire artifact provides the Cartesian coordinates of the guidewire artifact within the imaging data set of the image. Using the Cartesian coordinates of the guidewire artifact, data representing the imaging surface in one or more of the other images sharing the same Cartesian coordinates can be obtained and used to replace the guidewire artifact.

Any method for detecting a guidewire artifact within an image can be used. For example, a guidewire can be detected using principal component analysis, such as a technique described in co-pending application entitled "Automatic Stent Detection in Intravascular Images," Begin et al. To detect a guidewire using principal component analysis, a set of pre-defined images that are known to display a guidewire are generated to train a processor to identify or recognize the guidewire in images where the guidewire location is unknown. After a training set of the predefined images is generated, the principal components for the set can be computed to create an object space for the guidewire. By projecting an input image with an unknown guidewire location onto the object space, the guidewire can be detected within the input image. In another example, a guidewire artifact can be detected within an input image by generating a training set of images of a guidewire, where each image is defined by one or more features. A covariance matrix can be computed for a feature within each pre-defined image of the training set. The covariance for a feature within the input image can be calculated and compared to the covariances of the training set. From the comparison, the guidewire can be detected within the input image.

A further exemplary method for detection of a guidewire artifact utilizes the opaque nature of the guidewire by employing an edge detector or ridge detector as known in the art to detect the guidewire shadow artifact projected by a guidewire. Because the guidewires are often made of light-blocking material such as metal, the shadow in the surrounding tissue has a sharp edge in which the transition length is approximately equal to the lateral resolution of the imaging system. A shadow can be viewed as the inverted ridge (valley) amid surrounding tissues because it is usually the local intensity minima and it is substantially vertically oriented. Ridge-detecting filters are applied to the original image to obtain the ridge intensity level map. The ridge intensity at each scan or horizontal location is averaged in the vertical direction to generate the ridge intensity profile image or map. The average intensities of this map are then graphed and the peaks in the graph are detected. The peaks correspond to the location of the centers of the guidewire artifacts. The guidewire artifact can also be detected directly by using its bright ridgelike features. Information useful in implementing the ridge detector method exemplified may be found, for example, in Xu, U.S. Patent Publication No. 2010/0094127.

Another technique for detecting guidewire artifacts includes first detecting the lumen border and then detecting the artifact based on the intensity of pixels along the lumen border. This technique is described in detail in relation to stent strut shadows, in Unal et al., U.S. Patent No. 7,801,343. Detection of the lumen boundary may be accomplished, for example, by evolving a geometric shape, such as an ellipse, using a region-based algorithm technique, a geodesic boundary-based algorithm technique or a combination of the two techniques. The intensities of the pixels along lumen border are then used to detect the presence and location of the guidewire shadow in the image.

An additional suitable technique for detecting the guidewire artifact is described in Kimmel et al., U.S. Patent No. 7,397,935. This method examines each A-scan within a rotation spanning 360 degrees to determine which of the a-scans includes the guidewire artifact and/or shadow artifact. For example, a guidewire artifact 202 can be detected across A-scans by comparing the brightness of the brightest portion of data in an A-scan and a standard deviation of the brightness in the rest of the A-scan. A-scans that include a guidewire have a maximum difference between the brightest portion of data at the bottom of the A-scan and the standard deviation of brightness. Information useful in implementing this exemplary method may be found, for example, in Kimmel et al. U.S. Patent No. 7,397,935.

After the position of the guidewire artifact is known, the data representing the guidewire artifact in one image is replaced with data representing the imaging surface in another imaging image. The data representing the imaging surface can be taken from one or more other images. In certain examples, the resulting image with the guidewire artifact removed is then interpolated to enhance image quality using any method of interpolation known in the art. Suitable interpolation techniques include, for example, liner interpolation, cubic interpolation, and nearest neighbor interpolation, and are described in Bankman, Handbook of Medical Imaging: Processing and Analysis (2000).

In certain aspects, the acquired images may have more than one guidewire artifact present. For example, combined OCT and ultrasound imaging techniques may have a guidewire for the OCT imaging catheter and a guidewire for the ultrasound imaging catheter. In order to remove multiple guidewires present in an image, embodiments of the example are repeated for each guidewire. For example, removal of multiple guidewires is accomplished by acquiring images of an imaging surface having two or guidewires moved to different locations, registering the two or more images, detecting the guidewires in one or more images, replacing the detected guidewires in one image with data representing the imaging surface at the same location in one or more other images.

In some embodiments, a device of the invention includes an OCT imaging system and obtains a three-dimensional data set through the operation of OCT imaging hardware. In some embodiments, a device of the invention is a computer device such as a laptop, desktop, or tablet computer, and obtains a three-dimensional data set by retrieving it from a tangible storage medium, such as a disk drive on a server using a network or as an email attachment.

Methods of the examples can be performed using software, hardware, firmware, hardwiring, or combinations of any of these. Features implementing functions can also be physically located at various positions, including being distributed such that portions of functions are implemented at different physical locations (e.g., imaging apparatus in one room and host workstation in another, or in separate buildings, for example, with wireless or wired connections).

In some embodiments, a user interacts with a visual interface to view images from the imaging system. Input from a user (e.g., parameters or a selection) are received by a processor in an electronic device. The selection can be rendered into a visible display. An exemplary system including an electronic device is illustrated in FIG. 20.

## Claims

1. A device (400) for imaging tissue comprising:
an image capture unit configured to capture three-dimensional images inside of the tissue (201);
wherein the image capture unit is configured to capture a first image;
an electronic device comprising a memory coupled to one or more processors and configured to:
display the first image;
receive from an operator a selection of points from within the first image;
establish a boundary of a region of interest corresponding to the selected points; and
capture a three-dimensional image of the tissue within the designated boundary as a second image having a higher resolution than the first image,
wherein the second image includes only the region;
display the second image;
wherein the first and second image each comprise: i) a planar image along a vessel as an image longitudinal display, and ii) a tomographic view as a cross-sectional view of a vessel;
wherein the image capture unit is configured to capture the first image and the second three-dimensional image using sound waves or light waves; and
wherein the image capture unit comprises an intravascular ultrasound or OCT device.

2. The device of claim 1, further configured to capture the three-dimensional image by moving an image capture device along a line.

3. The device of claim 1, wherein receiving the selection of points comprises receiving computer input designating a set of pixels in the image.

4. The device of claim 1, further configured to render a graphical user interface to receive the selection of points.

5. The device of claim 1, further comprising a computer pointing device configured to be used in receiving the selection of points.

6. The device of claim 1, further comprising an intralumen catheter and further wherein the established boundary comprises start and stop points for a translation of the intralumen catheter.

7. The device of claim 1, further comprising an OCT catheter.

## Patentansprüche

1. Vorrichtung (400) zur Gewebebildgebung, die Folgendes umfasst:
eine Bildaufzeichnungseinheit, die dazu konfiguriert ist, dreidimensionale Bilder innerhalb des Gewebes (201) aufzuzeichnen;
wobei die Bildaufzeichnungseinheit dazu konfiguriert ist, ein erstes Bild aufzunehmen;
eine elektronische Vorrichtung, die einen Speicher umfasst, der mit einem oder mehreren Prozessoren gekoppelt und dazu konfiguriert ist:
das erste Bild anzuzeigen;
von einem Bediener eine Punkteauswahl aus innerhalb des ersten Bildes zu empfangen;
eine Grenze eines Interessensbereichs, der den ausgewählten Punkten entspricht, zu ermitteln;
und
ein dreidimensionales Bild des Gewebes innerhalb der bezeichneten Grenze als ein zweites Bild, das eine höhere Auflösung aufweist als das erste Bild, aufzunehmen, wobei das zweite Bild nur den Bereich beinhaltet;
das zweite Bild anzuzeigen; wobei das erste und das zweite Bild jeweils Folgendes umfassen:
i) ein planares Bild entlang eines Gefäßes als eine Bildlängsanzeige; und ii) eine tomographische Ansicht als eine Querschnittansicht eines Gefäßes;
wobei die Bildaufzeichnungseinheit dazu konfiguriert ist, das erste Bild und das zweite dreidimensionale Bild unter Verwenden von Schallwellen oder Lichtwellen aufzuzeichnen;
und wobei die Bildaufzeichnungseinheit eine intravaskuläre Ultraschall- oder OCT-Vorrichtung umfasst.

2. Vorrichtung nach Anspruch 1, die weiter dazu konfiguriert ist, das dreidimensionale Bild durch Bewegen einer Bildaufzeichnungsvorrichtung entlang einer Linie aufzuzeichnen.

3. Vorrichtung nach Anspruch 1, wobei das Empfangen der Punkteauswahl das Empfangen von Computereingabe, die einen Satz von Pixeln in dem Bild bezeichnet, umfasst.

4. Vorrichtung nach Anspruch 1, die weiter dazu konfiguriert ist, eine grafische Benutzeroberfläche zum Empfangen der Punkteauswahl zu rendern.

5. Vorrichtung nach Anspruch 1, die weiter eine Computerzeigevorrichtung umfasst, die dazu konfiguriert ist, beim Empfangen der Punkteauswahl verwendet zu werden.

6. Vorrichtung nach Anspruch 1, die weiter einen Intralumen-Katheter umfasst, und wobei die ermittelte Grenze weiter Start- und Stopppunkte für eine Verschiebung des Intralumen-Katheters umfasst.

7. Vorrichtung nach Anspruch 1, die weiter einen OCT-Katheter umfasst.

## Revendications

1. Une méthode (400) pour l'imagerie d'un tissu comprend:
Une unité de capture d'images configurée pour capturer des images tridimensionnelles à l'intérieur du tissu (201) ;
où l'unité de capture d'image est configurée pour capturer une première image; un dispositif électronique comprend une mémoire couplée à un ou plusieurs processeurs et est configuré pour:
afficher la première image;
recevoir d'un opérateur une sélection de points à partir de la première image;
établir une limite d'une région d'intérêt correspondant aux points sélectionnés; et
capturer une image tridimensionnelle du tissu à l'intérieur de la limite désignée comme seconde image ayant une meilleure résolution que la première image, où la seconde image inclue seulement la région;
afficher la seconde image;
où la première et seconde image comprend chacune: i) une image plane le long d'un vaisseau comme un affichage longitudinal de l'image, et ii) une vue tomographique comme une vue en coupe d'un vaisseau;
où l'unité de capture d'image est configurée pour capturer la première image et la seconde image tridimensionnelle en utilisant des ondes sonores ou lumineuses; et
où l'unité de capture d'image comprend une échographie intravasculaire ou un dispositif de tomographie en cohérence optique TCO.

2. Le dispositif de la revendication 1 est également configuré pour capturer une image tridimensionnelle en déplaçant le dispositif de capture d'image le long d'une ligne.

3. Le dispositif de la revendication 1, où la réception d'une sélection de points comprend la réception d'une saisie informatique désignant un ensemble de pixels dans l'image.

4. Le dispositif de la revendication 1, est également configuré pour rendre une interface utilisateur graphique pour recevoir une sélection de points.

5. Le dispositif de la revendication 1 comprend également un dispositif de pointage configuré pour être utilisé dans la sélection de points.

6. Le dispositif de la revendication 1 comprend également un cathéter intraluminal et en outre la limite comprend des points de départ et d'arrêt pour une translation du cathéter intraluminal.

7. Le dispositif de la revendication 1 comprend également un cathéter de TCO.
